# EUROPEAN PATENT APPLICATION

(11) **EP 1 980 268 A2**
(43) Date of publication of application: **15.10.2008**
(21) Application number: 07015810.0
(22) Date of filing: 12.04.2002
(51) Int. Cl.: A61K 39/145, A61K 39/295, G01N 33/569, C12N 5/06, A61P 37/06

(54) **Vaccination methods against avian influenza virus**

(62) Divisional of application: 02722692.7
(71) Applicant: Capua, Ilaria, 35027 Noventa Padovana (IT); Marangon, Stefano, 30174 Mestre (IT); Cattoli, Giovanni, 35100 Padova (IT)
(72) Inventor: Capua, Ilaria, 35027 Noventa Padovana (IT); Marangon, Stefano, 30174 Mestre (IT); Cattoli, Giovanni, 35100 Padova (IT)
(74) Representative: Smith, Stephen Edward

(57) **Abstract**

The inventions provides the use of an inactivated avian influenza virus in the preparation of a medicament for protecting against infection with avian influenza virus, wherein the inactivated virus is characterized by the same subtype of viral haemagglutinin and a different subtype of neuraminidase compared to the infectious virus against which protection is sought.

## Description

### Field of the invention

The present invention is in the field of zooprophylaxis actions that can be adopted to control and prevent high and low pathogenicity influenza epidemics from spreading.

In greater detail, the subject invention is designed to be advantageously employed in the avicultural field for controlling the shedding of avian influenza, especially by means of an effective discrimination of the animals that have contracted an influenza condition from the non-infected ones which have been immunized by means of a vaccination that has been set-up with heterologous virus strains. The invention consequently allows the evolution of the viral infection to be monitored in a population of animals that have been vaccinated with heterologous virus strain.

For this purpose, objects of the present invention are: a purified subfragment with genomic sequence coding for a neuroaminidase NAy protein of viral origin, in particular of the NA1 subtype; a recombinant antigen coded for by such a genomic sequence, said recombinant antigen being possibly employed for recognising antibodies that are produced in the course of influenza infection as infection markers; a process for expressing said recombinant antigen; a diagnostic method for detecting anti-NA antibodies of a specific influenza infection HxNy, and in particular for discriminating the infected animals from the vaccinated ones; a vaccination process that is particularly indicated for keeping under a tight control the development of influenza epidemics with discrimination of the infected individuals from the vaccinated ones; as well as a diagnostic kit that can be used as an easy-to-use tool for allowing such detection and discrimination.

### Background Art

It is well known that avian influenza (AI) is a highly infectious disease among birds, potentially causing considerable economical damage to avicultural breeders.

Consequently, in the avicultural circle, and inside the competent institutional organisms as well, there is strongly perceived the need for the development of effective prophylactic strategies to control and prevent the avian influenza epidemics, whether in highly pathogenic form (HPAI), or in lightly pathogenic form (LPAI) .

In greater detail, the influenza viruses are known to be classifiable in the various A, B, C typologies, according to the group antigen the viruses carry. The influenza viruses of the A, B, C types can be distinguished from one another on the basis of the antigen differences that can be found in the viral nucleocapsid (NP) and matrix (M) proteins. In particular, the A-type influenza viruses can moreover be classified in subtypes on the basis of antigen differences in the haemoagglutinin (HA) and neuroaminidase (NA) molecules. Presently 9 subtypes of the neuroaminidase NA proteins, that are indicated with NA1 to NA 9, and 15 different subtypes of the serum haemoagglutinin HA proteins, that are indicated with HA 1 to HA 15, usually respectively associated to the 15 viral subtypes, that are indicated with H 1 to H 15, are known. In birds viruses carrying any HA and NA subtype can be isolated.

HA is a viral surface glycoprotein comprising approximately 560 amino acids and representing 25% of the total virus protein. It is chiefly responsible of adhesion of the viral particle to the host cell and of its penetration into the latter in the early stages of the infection. Haemoagglutinin, among the viral proteins, is the one that is most subject to post-translational rearrangements. After the synthesis thereof has been completed, the molecule follows the exocytotic pathway of the host cell, in the course of which HA is folded, assembled in trimers and glycosylated. Finally it is cleaved into two subunits H1 and H2; this cleavage is the key step in the activation of the molecule and in the acquisition of the infective capacity by the virion.

It is well known, in fact, that the different composition of the cleavage site, concerning the basic amino acid residues, translates into the capacity of the AI virus to produce localized scarcely, or not at all, symptomatic infections, or, vice versa, generalized infections having a lethal outcome for many avicultural species. It has therefore been suggested that this fact might influenza the organ-tropism, the host specificity, as well as the pathogenicity of the virus. Concerning the pathogenicity of the virus, strains with multibase-site HA find proteases that cleave the H0 molecule, in the active form H1 and H2 in several cellular types thus giving rise to multiple infection cycles with a massive production of infectious viral particles and causing a generalization of the infections in all of the districts within a short time period (HPAI strains). The infection will consequently turn out to have an acute-hyperacute course, with very high mortality.

Neuroaminidase (NA) represents the second membrane glycoprotein of the influenza A viruses. it is coded for by a gene (segment 6 of the virus RNA) of 1413 nucleotide length that codes for a 413 amino acid peptide. This protein has at least two important functions: destruction of the cellular receptor for the viral haemoagglutinin by cleaving between the sialic acid molecule and the haemoagglutinin itself. In this way it is supposed to possibly ease the liberation of the viral progeny by preventing the newly formed viral particles from accumulating along the cell membrane, as well as promoting the transportation of the virus through the mucus that is present on the mucosal surface. NA moreover represents an important antigen determinant that is subject to antigenic variations.

The host that has been infected by an influenza virus produces a remarkable antibody response raised against this antigen but, differently from what occurs with haemoagglutinin, these antibodies are not primarily neutralizing ones and, by themselves, would not be capable to protect the host against new infections.

It is clear that the above-mentioned epidemiological control strategies are based on the possibility to detect the infection caused by influenza viruses after it has occurred. Within said strategies, therefore, the identification of the viral strains for a possible setting up of "ad-hoc" vaccines, the survey of the immunity degree of an animal population before and during an influenza infection, as well as the possibility of reliably diagnosing an influenza infection during epidemics take a special importance.

A well known control tool, that is widely employed in certain countries, such as Mexico and Pakistan, in the presence of serious epidemic events of avian influenza, is still represented by vaccination.

This technique notoriously exploits the ability of certain internal or membrane virus proteins that are contained in the vaccine to induce an immunization response in the body of the vaccinated animal. Such response is expressed through the production of antibodies against the specific virus strains that are responsible of the corresponding pathologic influenza conditions.

Notoriously, the studies aiming at setting up vaccines for the prophylaxis of influenza infections have mostly focused onto the use of the haemoagglutinin HA protein. This latter, in fact, by being implied in the absorption and in the penetration of influenza virus into the host cells is the primary target of the neutralizing antibodies.

It is well known that vaccines can be of the conventional type or of the engineered type.

More particularly, the first ones are prepared from the natural, suitably inactivated epidemical (field) virus, which stimulates' in the immunized subject the production antibodies that are identical with those generated as a consequence of the natural infection. On the contrary, it is well-known that the second ones, of the engineered type, are obtained through genetic engineering techniques or through virus mutations induced with serial passages in the laboratory.

The main problem in the application of the vaccination policies is the discrimination between vaccinated subjects (which have not been in contact with the field virus) and the infected ones.

In the specific case of avian influenza, this has been included in List A of the Office International des Epizotes (OIE), which list comprises highly shedding animal diseases, and vaccination against it has been forbidden (with any kind of vaccine) in the European Union (EU) countries in order to avoid any possible interference with serum monitoring and eradication plans.

The avian influenza control policies that exploit the use of conventional vaccines have exhibited in practice several limits, such that presently in many countries their use is only allowed in exceptional events of enormous proportion epidemics.

In fact, the main limit of such known vaccination techniques can be traced back to the scarce possibility to carry out effective controls on the possible evolution of the live vaccination virus strain within the animal population. As a matter of fact, the vaccination prevents mortality and clinical symptoms, whilst it does not prevent the "healthy carrier" condition. As a consequence, vaccinated subjects that have subsequently been infected can represent the source of a further diffusion of infection, in opposition to the target of containing and eradicating the infection. It appears therefore to be of a fundamental importance to know, within a vaccination campaign, the epidemiological reality so as to be capable to discriminate the subjects that are serum-positive because they have been vaccinated from the subjects that are serum-positive because they have had a contact with the field virus.

In the same way, the exploitation of engineered vaccines within avian influenza control policies has met several hurdles.

Such vaccines are envisaged in order to fight with an ever increasing effectiveness the conditions that are associated with the different subtypes of influenza viruses. They are developed with advanced genetic engineering techniques that give way to the production of more and more sophisticated recombinant vaccines comprising genomic sequences that are capable of coding for specific viral proteins that are carried by suitable vectors.

Vaccines of this kind provide for example for the expression of the haemoagglutinin antigen HA5 or HA7 that is inserted and expressed in the fowl pox virus in order to raise a protective immune response to the H5 and H7 virus subtypes.

The problem of controlling the infection through indirect prophylaxis in certain conditions is thus unavoidable. In particular, the infections caused by the influenza viruses of the subtype H5 or H7 have been responsible in the past of the most serious highly pathogenic avian influenza epidemics with devastating effects on the intensive chicken and turkey breeding farms in various parts of the world, such as Pennsylvania in 1984, with more than 17 million birds died, as well as more recently in Mexico, Pakistan and northern Italy (14 million birds died).

The vaccines obtained from genetically modified live viruses have difficulties in registration within the European Union because they are considered potentially dangerous from the point of view of environment impact.

Indeed, the use of live genetically modified viruses on the animal populations causes a dispersion of the virus into the environment, and such a genetically modified organism might interact, from the genetical point of view, with higher organisms or with microorganisms in an uncontrollable manner.

In order to allow a greater control of the sheddable diseases of the animals, the concept of "marker vaccine" has found its path in recent years, that is of engineered vaccines that are characterized by the presence, the absence or the mutation of a specific protein, while maintaining sufficient cross-protection capacities against the field virus.

The employment of the present-day marker vaccines exhibits however the same limits that have been highlighted above concerning the present vaccination techniques.

### Disclosure of the invention

In this situation, the main object of the present invention is consequently to overcome the limits and draw-backs that are associated with the previously known prophylaxis techniques for the control of high and low pathogenicity influenza epidemics, especially in the avicultural field, by providing a strategy that would allow the animals which have contracted an influenza virus to be easily recognized and distinguished from those individuals that are serum-positive as a consequence of vaccination.

A particular object of the present invention is providing a purified nucleic acid fragment (subfragment) according to claim 1.

A further object of the present invention is to provide a process for the expression of a neuroaminidase protein Nay as a marker antigen.

A further object of the present invention is to provide a recombinant antigen that can be obtained through the expression of said subfragment.

A further object of the present invention is to provide a diagnostic test based on said recombinant antigen and capable of discriminating infected animals from vaccinated ones.

A still further object of the present invention is to provide a process for the vaccination of avicultural animals against infections caused by avian influenza.

### Brief description of the drawings

The technical features of the present invention, according to said objects, can be clearly found out from the contents of the attached claims, and the advantages of the same will be more evident from the following detailed description that follows a preferred, non exclusive, embodiment of the present invention, provided with reference to the accompanying drawing,-in which:
FIG 1 is a micrograph showing the result of an indirect immune fluorescence test performed on H7N1-positive serum.
FIG 2 is a micrograph showing the result of an indirect immune fluorescence test performed on H7N3-positive serum.

### Detailed description of a preferred embodiment example

Hereinafter the present invention has been described in detail with particular reference to a case of type A avian influenza caused by a virus strain with subtype H7N1. However it is to be understood that the same invention can also be adopted for the development of strategies for controlling and preventing the diffusion of high and low pathogenicity influenza epidemics deriving from any HxNy virus strain, wherein x and y point out any subtype of H and N, respectively.

The inventive idea is particularly to make use of inactivated conventional vaccines, that are easily produced, low cost vaccines of established effectiveness, containing an influenza strain of the same subtype as far as HA is concerned, but heterologous as far as the NA subtype is concerned.

Such a choice is based on the knowledge that the Hax protein of haemoagglutinin is responsible of the production of antibodies, and that consequently any Hx virus is capable of stimulating the synthesis of protective antibodies, whilst heterologous neuroaminidase is exploited as a natural marker.

In this way, the animals can be vaccinated and protected against an infection caused by an influenza virus that belongs to the same haemoagglutinating subtype, while allowing the vaccinated animals to be discriminated from the infected ones by determining the specific anti-NA antibodies.

With reference to the specific case under examination, it will be highlighted that the clinical protection can be warranted through a vaccine that has been set up from a virus strain with homologous haemoagglutinin, but heterologous neuroaminidase. In the specific case, the experimental tests have been carried out with the use of a virus strain H7N3 as a constituent of the vaccine, while the field (or challenge) virus exhibits an antigenic conformation H7N1. Therefore, on the one hand the homologous group H7 provides the clinical protection, on the other hand the detection of the infected animals can be based on the fact that the infected animals develop antibodies against protein N1, while the vaccinated animals develop antibodies against the heterologous neuroaminidase N3, but non against N1, unless they also are infected.

In order to do this, it is on the one hand necessary to provide a diagnostic method that is in a measure to detect said specific antibodies anti-NA (in this case N1), and on the other hand to prepare a natural vaccine that is capable to maintain a satisfying cross-protection capacity towards specific strains of avian influenza, while being distinguished from the virus by a different neuroaminidase.

The preparation of the method requires in turn that an antigen is set-up which can be represented by a Nay protein or by any vector that contains a genomic sequence coding for neuroaminidase protein Nay in an essentially non-modified state as compared with that of the specific avian influenza virus strain HxNy.

In case of avian influenza H7N1, the antigen according to the invention is prepared by previously extracting the virus RNA from the virus strain A/ty/Italy/4426/V00/ H7N1 LPAI and then amplifying the virus RNA segment coding for the neuroaminidase protein NA1.

The RNA sample is to be first converted into cDNA in order to possibly provide the required template to the temperature-stable polymerase.

The RNA segment is therefore first back-transcribed by reverse transcriptase RT.

After this starting V step, the procedure follows the described cycles of the PCR reaction, under amplification of the interested sequence as a DNA molecule. The quality and purity of the initial RNA template is determinant for the success of RT-PCR.

The PCR then allows the specific cDNA sequence, and only this one, to be amplified. Therefore, after denaturing the cDNA, the single strands are caused to anneal (renaturate) In the presence of an excess of two oligonucleotides (primers) representing the boundary sequences of the region to be amplified which, according to the present invention, for the cDNA-polymerase of the neuroaminidase protein NA1 consist of the forward primer 5'- GCG CGC GGC CGC CAG GAG TTT AAA ATG AAT CCA AAT C -3' (SEQ. I.D. No. 1) and of the reverse primer 5'- GCG CGC GGC CGC CTA CTT GTC AAT GGT GAA TGG C -3' (SEQ. I.D. 2). In the sequence of such primers a specific length is inserted that is recognised by the restriction enzyme Not I. The amplification product that will be obtained with said primers will then possibly be digested by enzyme NotI, thus making the cloning process of the hereinafter described gene possible.

Owing to the molar excess of the oligonucleotides, the cDNA strands will anneal almost exclusively with the two oligonucleotides, rather then with one another. The products of the first reaction will then function as templates for the synthesis of the new strands. The whole cycle is repeated again until the amplified helices will be so many as to anneal with one another rather then with the two oligonucleotides. This saturation is reached after a remarkable number of amplification cycles. In practice the original fragment will thus be amplified millions of times.

The amplification of the cDNA derived from said RNA genomic sequence brings, in the case under examination, to have a purified subfragment (amplification product obtained by means of RT-PCR) preferably having a length of about 1.4 Kb.

At this point the cDNA of the thus obtained gene is gel-purified and sequenced to confirm the identity of the amplification.

Cloning of the gene coding for the NA and production of recombinant NA allow relatively large amounts of antigen to be obtained for use in the diagnostic method and especially, as shown hereinafter, in serum tests.

Such an antigen, in case it is represented by protein N1, obviously contains the solve protein of interest, thereby avoiding the problem of the possible contamination with other virus proteins, especially the HA protein, that would cause the occurrence of cross-reactions.

Moreover, as outlined above, it is necessary that this recombinant protein maintains as little changed as possible the antigen characteristics peculiar to the original peptide, said characteristics being essentially conferred by the amino acid sequence, by the three-dimensional structure of the molecule and, very importantly, by its glycosilation.

According to a further feature of the present invention, the most suitable expression system to obtain a protein that maintains said characteristics has turned out to be the eukaryote system in insect cells using as expression vectors the baculoviruses, i.e. DNA viruses that are capable of replicating only in arthropods (insects and crustaceans).

Since there is no evidence that these viruses can infect other invertebrates or higher organisms, such as plants or mammalians, their safeness is well-accepted, and the laboratory that is to produce the antigen with these expression systems does not have to apply extraordinary safety provisions, this being a further advantage from both practical and economical points of view.

Therefore, after having been amplified the above-described cDNA subfragment is advantageously expressed using a Bac-to-Bac® (Invitrogen, LifeTechnologies) in baculovirus vector expression process, said process being hereinafter summarized.

The cDNA is NotI-digested, ligated in a NotI-cleaved pFast-Bac donor plasmid and cloned in E.coli DH5α competent cells. E.coli colonies containing the correct insert are selected and the recombinant pFast-Bac is subsequently extracted. E.coli DH10Bac cells, containing the baculovirus shuttle vector (bacmid), are pFast-Bac transformed and the recombinant bacmid is isolated and used for transfection of Trichoplusia ni insect cells (HighFive® cells, Invitrogen, LifeTechnologies).

Recombinant baculovirus particles are collected from cell cultures after 72 hours and titrated by plaque assay. The expression of the gene of interest is confirmed by Western blot.

The thus obtained recombinant antigens is therefore represented by the recombinant glycoprotein neuroaminidase NA1 the genomic sequence of which is carried by a baculovirus and expressed in insect cells.

As hereinafter described, it is adapted to be advantageously employed within a diagnostic method for the detection of positiveness for an avian influenza viral infection with epidemic strain H7N1.

According to a further characteristic of the subject invention, after completing the antigen preparation step the discrimination step comprises a step for taking a biological fluid from the body of a vaccinated animal under diagnosis followed by a step of contacting the recombinant antigen with said biological fluid, with the subsequent detection of an antigen-antibody reaction by means of a test for detecting positiveness in case the subject is infected.

The detection test can advantageously consist of an indirect immune fluorescence, or immune peroxidase, or an ELISA test, either indirect or competitive, or a precipitation test, or any other quick test.

As quick tests, those tests are meant in which a detection system of varied nature (usually an enzymatic or otherwise colour test) provides evidence of the antigen-antibody reaction.

Such tests are usually constructed in such a way as to be usable on the field and do not require any special equipment.

An object of the present invention is moreover a vaccination process against avian influenza which, with reference to the hereinafter outlined exemplary case, is due to virus strain H7N1.

Such process comprises an initial step for the preparation of a natural vaccine (i.e. a vaccine derived from an inactivated natural virus), said vaccine being heterologous because it is characterized by the same subtype of viral haemoagglutinin HA7 and a different subtype of neuroaminidase NA3.

It is essential that this kind of vaccine should exhibit a satisfying cross-protection capacity against the specific field virus to which it is to raise an immunizing action while being distinguished therefrom by a different neuroaminidase.

In the subject case, we have observed a surprisingly effective cross-protection action of the heterologous H7N3 vaccine against virus H7N1.

Hereinafter the surprising results of cross-protection studies will be described, in which birds immunized with a vaccine containing the inactivated heterologous strain A/ck/Pakistan/95 H7N3 were infected with HPAI A/ty/Italy/4580/V99 H7N1.

In greater detail, the isolated highly pathogenic field virus HPAI A/ty/Italy/4580/V99 (H7N1) was used as the challenge virus, administered at a dosage of 10^{7,5} EID₅₀ by conjunctival instillation of 0.05 ml eye drops. The vaccine used was an inactivated oil-emulsion vaccine containing 0.3 ml/dose of A/ck/Pakistan/95 (H7N3) (commercially available as Fluvac^{®}, manufactured by Merial).

50 white leghorn SPF chickens were used for the challenge experiments and were divided randomly into 5 experimental groups. They were hatched and reared in isolation and fed with water and feed ad libitum. All birds except those in groups 4 and 5 were challenged at 6 weeks of age.
Group 1: 10 unvaccinated control birds, challenged at 6 weeks of age;
Group 2: 13 birds were vaccinated s/c with 0.3 ml of Fluvac^{®} at two and at four weeks of age;
Group 3: 13 birds were vaccinated s/c with 0.3 ml of Fluvac^{®} at three weeks of age;
Group 4: 11 unvaccinated, unchallenged controls;
Group 5: 5 unvaccinated "sentinel" birds, which were placed together with group 2, 2 hours post challenge. This experimental group of sentinel birds was introduced into the isolators to evaluate the transmission of virus from the vaccinated birds.

Following vaccine administration, and challenge, the birds were observed daily for clinical signs and mortality throughout the duration of the experiment. Prior to challenge all birds were bled from the wing vein for the detection of antibodies to H7 by the haemagglutination inhibition test using 4 haemagglutinating units (CEC, 1992).

To evaluate the shedding levels, biological material was swabbed from the birds on days 2, 4, 7, 10 and 14 (with tracheal and cloacal swabs) and the swabs processed for the virus isolation at limiting dilutions (10⁻¹ to 10⁻⁷ ) as described (CEC, 1992).

In order to investigate the colonisation of target organs by the challenge virus, two clinically healthy birds from groups 2 and 3 were sacrificed on days 2 and 4 post-challenge and necropsied. Their organs were then selected (brain, cerebellum, liver, spleen, heart, trachea, lung, pancreas, kidney, adrenal gland, duodenum, caecal tonsil and bursa of Fabricius) and were then processed for histopathology and immunohistochemistry (IHC) for antigen detection as described. Samples of pectoral and thigh muscles were also collected from these birds in order to evaluate the presence of the virus in the muscles of vaccinated healthy birds by conventional virus isolation methods (CEC, 1992).

Similar organ samples were collected from all birds dying post challenge to evaluate the virus distribution in target organs. In addition, pooled trachea and lung, intestine and kidney samples and the specimens obtained from pectoral and thigh muscles were also processed for virus isolation (CEC, 1992) from all the birds that died throughout the duration of the experiment.

Serum samples were collected from surviving birds 2 and 6 weeks post challenge in order to evaluate the titre to H7 by means of the HI test (CEC, 1992).

The results of the HI test performed on the experimental birds prior to challenge are presented in Table 1. As expected, the birds vaccinated twice exhibited higher serological titres compared to those vaccinated once. In groups 2 and 3 2/13 and 5/13 birds respectively had titres of lower than 1:16, which is considered the cut-off limit for positivity. All the birds belonging to group 4 (unvaccinated, unchallenged) exhibited negative results in the HI assay.

None of the birds belonging to group 4 ever exhibited any clinical sign of disease.

All the unvaccinated challenged birds (Group 1) exhibited within 4 days post-infection the typical signs and lesions of HPAI. Virus was recovered by isolation from the pooled lungs and tracheas and from the intestines of all these birds and from both pectoral and thigh muscle samples collected from 8/10 birds. 10/10 cloacal swabs and 8/10 tracheal swabs collected from challenged unvaccinated birds were positive by virus isolation on day 2 post-infection at titres ranging from 10² to 10⁴ EID₅₀ per ml. One bird in group 2 and one in group 3 died on days 4 and 6 post-challenge respectively. These birds exhibited serological titres of <1:2 and 1:4 respectively prior to challenge. The other birds appeared healthy throughout the duration of the experiment. The sentinel birds that had been introduced in the isolators to evaluate the "in vivo" shedding of group 2 started dying on day 8 post-introduction. All the sentinels died by day 13 with the exception of one sentinel that survived and was kept 2 months after the termination of the experiment. At no time did this bird show any clinical signs and failed to seroconvert to H7.

The results of the virological investigations performed on the organs, tissues and cloacal and tracheal swabs collected from all the dead or sacrificed birds are presented in Tables 2 and 3.

On examining the data it appears that virus shedding was detected by virus isolation in the samples collected from group 3 (vaccinated once) to a maximum of 10⁴ EID₅₀ per ml (data not shown) and up to day 7 post-inoculation. Virus was not recovered from any bird in group 2, except from the serum negative bird that died following challenge, on day 4 post-infection.

Virus was not recovered from any of the muscle samples collected from the clinically healthy vaccinated birds sacrificed on days 2 and 4 post-challenge.

All the controls on unvaccinated birds showed post mortem and histopathological lesions typical of avian influenza. Additionally, viral antigen was detected in the brain, cerebellum, liver, spleen, heart, trachea, lung, pancreas, kidney, adrenal gland, duodenum, caecal tonsil, and Bursa of Fabricius of the birds belonging to groups 1 and 5 (unvaccinated controls and sentinels for in vivo shedding).

The organs that exhibited strongest IHC positivity were brain, kidney, heart and pancreas.

Viral antigen was not detected in any of the sacrificed vaccinated birds belonging to groups 2 and 3. In contrast viral antigen was detected in the two birds that died 4 and 6 days post challenge. However, in these birds only mild positivity was observed in IHC and the viral distribution was restricted to a few foci in the brain, heart, kidney and pancreas.

The results of the serological investigation in the vaccinated birds surviving challenge are presented in Table 2. Geometric mean HI titres (GMT) were significantly higher in the birds vaccinated once than in the birds vaccinated twice. This could possibly represent the effect of a higher degree of replication of the challenge virus in the birds with lower HI titres prior to challenge (Group 3).

Heterologous H7N3 vaccine induced clinical protection levels of 93% in each of the vaccination schemes used and prevented viraemia in SPF (specific pathogen-free) birds when challenged with the HPAI H7N1 strain A/ty/Italy/4580/V99. Moreover, the number of birds shedding infection turned out to be reduced in the vaccinated bird groups when compared to the unvaccinated controls.

The results of this investigation supply experimental data on two of the essential issues that are frequently raised when approaching the topic of vaccination for avian influenza in poultry, namely the reduction of shedding and the presence of infectious virus in the muscle. Our experience confirmed that shedding does occur, although to a lesser extent than in the unvaccinated controls, and that, particularly in the birds that were vaccinated twice, the levels of shedding were so low that no virus was detected by conventional virology techniques, but only by the introduction of SPF sentinels. The absence of any positivity in IHC in the clinically healthy vaccinated birds, suggests that viraemia was not established and that possibly the shedding that was observed was a result of limited virus replication in the intestinal and respiratory tracts. Similarly, no virus was isolated from the breast or the thigh muscle of the clinically healthy vaccinated birds, this is in keeping with the absence of viraemia reported above.

The main the aims of enforcing a vaccination policy against avian influenza are to protect birds from clinical signs and viraemia and to reduce the amount of virus shed into the environment.

Thus it can be observed that altogether the cross-protection results have put in evidence a surprising protection induced by the heterologous vaccine H7N3 with both of the employed vaccination schemes, achieving a protection index of 93%.

The vaccination strategy that is the object of the present invention also provides for the possibility of discriminating whether the serological positivity is only induced by the vaccination, or a superinfection with field virus has occurred. Such a determination is carried out by means of the above-mentioned diagnostic method comprising the preparation and use for diagnostic purposes of an antigen according to the above-described process such that it contains the neuroaminidase protein NA1 of the field virus.

At this point, according to the detection method a biological fluid is taken from the body of a serum-positive animal individual, followed by challenging it with the recombinant antigen in order to be able to point out a possible positivity through an antigen-antibody reaction by means of a test for the detection of positivity.

The biological fluid to be tested is for example taken from an animal population that is at risk of infection comprising at least a group of vaccinated animal group.

Advantageously, however, as a rule there will be provided for the vaccination of the whole animal population.

In greater detail, in case an indirect immune fluorescence test is used as the detection test, such test being hereinafter referred to as iIFA test, the antigen is preferably prepared as hereinafter specified.

HighFive® cells were seeded on chamber slides (inoculum 250 µl/well) or in 96 micro-well plates (inoculum 50 µl/well) at a concentration of 0.9-1 x 10⁶ cells/ml in TC-100 culture medium supplemented with 10% foetal calf serum and 1% antibiotic solution, incubated at 28°C for 1 hour and then infected with recombinant bacmid at a multiplicity of infection (MOI) of 2. Infected cells expressing the recombinant N1 protein were fixed in acetone after 48 hours of incubation at 28°C and stored for up to 4 weeks at +4°C.

Serum samples to be tested were stored at -20°C and pre-diluted 1:250 in PBS, pH 7.4. 50 µl of pre-diluted test and control sera were dispensed in duplicate wells, incubated at 37°C for 45 minutes, washed 3 times for 5 minutes in 200 µl of PBS, incubated at 37°C for 45 minutes with 25 µl of FITC (fluoresceine isothiocyanate)-conjugated anti-chicken IgG immunoglobulins as the secondary antibody, and Evans blue (working dilution 1:500; stock solution 0.5%), and, after incubation, washed 3 times for 5 minutes in 200 µl of PBS. The wells were overlaid with 25 µl of glycerol in carbonate buffer, pH 9.6, cover slips were mounted onto chamber slides with the same buffer and read under an UV microscope.

The following controls were included in each plate/batch of chamber slides: an H7N1 positive serum obtained from experimentally infected SPF chickens (HI titre 1:128); an H7N1 positive serum obtained from experimentally infected turkeys (HI titre 1:16); a negative serum obtained from SPF chickens; a negative turkey serum obtained from a commercial turkey; an H7N3 positive serum obtained from H7N3 vaccinated SPF chickens (HI titre 1:1024); an H7N3 positive serum obtained from H7N3 vaccinated turkeys (HI titre 1:128). All control sera were obtained from SPF or commercial birds hatched and reared in isolation.

A total of 608 turkey field sera were used to validate the test. Of these, 107 were of H7N1 animals infected naturally (HI titre ≥1:16); 140 were obtained from H7 negative animals; and 361 were obtained from H7N3 vaccinated birds (titre ≥1:16). The agreement between the HI result and the iIFA was assessed by statistical analysis (Kappa value), thus enabling the determination of the relative sensitivity and specificity.

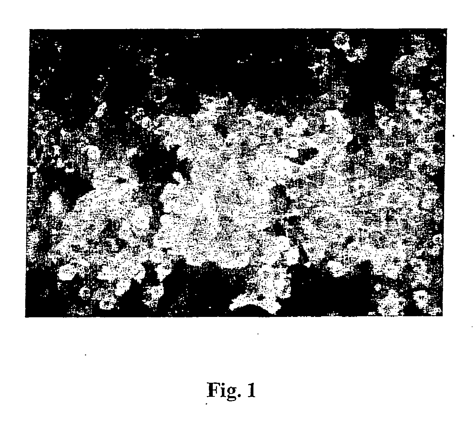

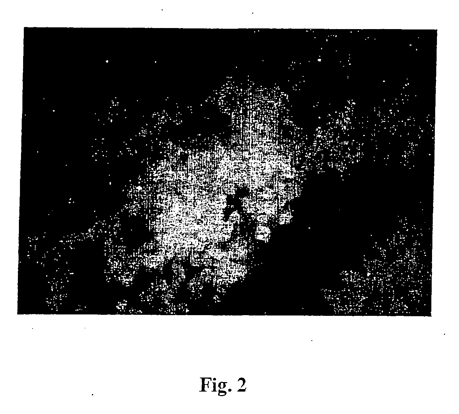

On the basis of the fluorescence observed with the control sera, the samples were scored as follows: positive samples = fluorescence limited to 50-80% of the monolayer corresponding to foci of cytopathic effect (CPE), the fluorescence is bright and mainly located in the proximity of the cell membrane (Fig 1) ; negative samples = absence of fluorescence or faint yellow-greenish background present in all cell compartments, and present on 100% of the monolayer (Fig.2); doubtful sample = granular or pale fluorescence on the cell membrane of the majority of the cells.

The results of the test validation are presented in Table 4. The statistical analysis performed indicated a Kappa value of 0.93, which is defined as "almost perfect agreement". The relative sensitivity and specificity of the test were calculated as 98.1 and of 95.7 %, respectively (CI_{95%}= 92.4; 99.1).

The development and validation of the N1-N3 discriminatory iIFA test indicates that this test can be considered in almost perfect agreement with the HI test. The levels of relative sensitivity and specificity can be considered more than satisfactory in distinguishing the N1-infected from N3-vaccinated birds.

Differently from what is described above, said detection method can provide for the use of less labour-intensive detection tests, such as, in particular, indirect ELISA, competitive ELISA, immunoperoxidase, precipitation tests, or other quick tests.

In the case of indirect ELISA, the antigen, that is the recombinant baculovirus (or any purified protein derived therefrom), is adsorbed onto a 96-well plate overnight at 4° C in carbonate-bicarbonate buffer, pH 9.6-9.8. The test serum is then successively dispensed into the plate at a predetermined dilution, and incubated at 37°C or at room temperature for 1 to 3 hours. After washing with a suitable buffer, the anti-species enzyme conjugate is dispensed, which is then incubated and thereafter washed as previously described. Thereafter the substrate is added, which will be capable to provide evidence of the presence of the peroxidase, if any.

The correspondence between antigen and antibody is manifested in a colour reaction due to the activity of the enzyme complex bound to the secondary anti-species antibody.

In this test the positive sample will be coloured, the negative sample being instead colourless.

In the case of competitive ELISA, the recombinant baculovirus (or any purified protein derived therefrom) is adsorbed onto a plate in carbonate-bicarbonate buffer, pH 9.6-9.8. A monoclonal or polyclonal serum is used, provided it is antigen-specific. The test serum is dispensed into the plate, and concurrently or after an incubation step the pertinent serum at a predetermined dilution is added. After washing with a suitable buffer, the anti-species enzyme conjugate is dispensed, which in this case is for providing evidence of the antibodies of the pertinent serum, and the plate is incubated and thereafter washed, as described previously. Thereafter the substrate is added, which will be capable to provide evidence of the presence of the peroxidase, if any.

The correspondence between antigen and antibody is manifested in a colour reaction due to the activity of the enzyme complex bound to the secondary anti-species antibody.

In this test the positive sample will be colourless, the negative sample will instead be coloured.

The immunoperoxidase test is similar to the immunofluorescence test, the difference being that the secondary antibody is conjugated to peroxidase instead of fluoresceine; the reading is therefore made under the optical microscope.

A cell substrate infected with recombinant baculovirus in this case is fixed, followed by dispensing into each well the suitably diluted test sera. The plate is incubated at 37°C or at room temperature for 1 to 3 hours. After washing with a proper buffer, the anti-species enzyme conjugate (peroxidase) is dispensed which is then incubated and subsequently washed as previously described. The substrate is thereafter added that will allow the presence of peroxidase (if any) to be detected, provided there is complementarity between antigen and antibody.

Positivity will be revealed by a specific coloration of the infected cells, which can be pointed out at the optical microscope.

The immune precipitation tests are based on the formation, in an inert support, of an insoluble complex that can be seen with the naked eye, from two soluble components, in this case the test serum and the recombinant neuroaminidase containing antigen.

In the case of quick tests, a colour-developing detection system of a varied nature, usually an enzyme-based one, provides the evidence of the antigen-antibody reaction. Such tests are generally constructed in such a way as to be usable on-the -field and do not require any special instruments.

In this case a drop of the test sample (serum) is added to an inert-material support in which the antigen is adsorbed. After a few minutes a drop of detection system plus substrate is then added. The positivity is indicated by one or several circumscribed colour stains on the inert substrate.

A further object of the present invention is moreover, advantageously, a diagnostic kit for the detection of the infection after it has occurred in a vaccinated animal population. It contains essentially a solid support of an inert material, a recombinant antigen resulting from the expression of said subfragment coding for the neuroaminidase protein Nay in a state that is essentially non-modified as compared with that of the specific avian influenza virus strain HxNy relative to which the positivity of the animal is to be checked.

The antigen is for example fixed by adsorption onto the latex sphere solid support, or it is adsorbed onto plastics solid supports particularly provided in the ELISA test.

There is also provided the use of reagent that is adapted to colorimetrically highlight the positivity to the viral infection in the presence of anti-Nay antibodies contained in a biological fluid extracted from an animal individual and added on top of the support.

The antigen production can be advantageously carried out in accordance to the example previously described in detail with reference to protein NA1.

The biological fluid to be tested is for example taken from individuals of an animal population that has been vaccinated with a heterologous vaccine characterized by the same subtype of virus haemoagglutinin Hax and a different subtype of neuroaminidase Naz (with z any neuroaminidase different from y). The kit thus set-up allows in any case a discrimination between infected individuals and the other serum-positive individuals to be established, thus preventing the presence of false positive results due to vaccination.

The present invention indicates that the use of an inactivated vaccine with haemoagglutinin of the same group and a heterologous neuroaminidase is a surprisingly effective solution to be followed for controlling avian influenza, as it allows on the one hand a good clinical protection to be obtained, and offers on the other hand the possibility of discriminating the vaccinated subjects from the infected ones.

Therefore, such a strategy can be applied, beyond the herein disclosed exemplary case under examination, to the control of infections caused by different subtypes, thereby allowing official health institutions and veterinarians to identify actually infected bird groups by discriminating between infected and vaccinated birds, which is the basis for any control strategy aiming at eradicating a viral disease.

The control strategy disclosed in the present invention suggests that this strategy might be applied to the control of both HPAI and LPAI infections all over the world, provided only one subtype at a time is circulating.

**Table 1. Serological response of birds to vaccination**

| **titres prior to challenge* Group** | **<1:16** | **≥1:16** | **≥1:32** | **≥1:64** | **≥1:128** | **≥1:256** | **≥1:512** | **GMT log₁₀** |
|---|---|---|---|---|---|---|---|---|
| **2 (vaccinated twice)** | 2/13 | 11/13 | 10113 | 7/13 | 4/13 | 2/13 | 1/13 | 45 |
| | | | | | | | | |
| **3 (vaccinated once)** | 5/13 | 8/13 | 5/13 | 3/13 | 2/13 | 1/13 | 0/13 | 19 |

| | | | | | | | | |
|---|---|---|---|---|---|---|---|---|
| * N° with titre / n° vaccinated | | | | | | | | |

**Table 2. Serological response and virus isolation attempts from vaccinated birds immunised with inactivated vaccine containing A/ck/Pakistan/95 (H7N3) virus and challenged with A/ty/Italy/4580/V99 (H7N1) virus.**

| **Virus isolation^{§}** | | | | | | | | |
|---|---|---|---|---|---|---|---|---|
| | ***GMT ^ log₁₀*** | | | | | | | |
| | **2 weeks** | **6 weeks** | **intestine** | **lung + trachea** | **kidney** | **pectoral** | **thigh** | **clinical signs** |
| **Group** | **p.c.** | **p.c.** | | | | **muscle** | **muscle** | |
| | | | | | | | | |
| **2 (vacc. twice)** | 108 | 75 | 1*/5 | 1*/5 | 1*/5 | 1*/5 | 1*/5 | 1*/13 |
| | | | | | | | | |
| **3 (vacc. once)** | 152 | 210 | 0/5 | 1*/5 | 1*/5 | 0/5 | 0/5 | 1*/13 |
| | | | | | | | | |
| **Control** | - | - | 10/10 | 10/10 | 10/10 | 8/10 | 8/10 | 10/10 |

| | | | | | | | | |
|---|---|---|---|---|---|---|---|---|
| ^{^} Geometric mean titre in HI test after challenge ^{§} Number of chickens positive/number of chickens analysed ^{•} Died of AI | | | | | | | | |

**Table 3. Results of virus isolation assays from cloacal and tracheal swabs of control and vaccinated birds immunised with inactivated vaccine containing A/ck/Pakistan/95 (H7N3) virus and challenged with A/ty/Italy/4580/V99 (H7N1) virus.**

| Virus recovery from | | | | | | | | | | |
|---|---|---|---|---|---|---|---|---|---|---|
| | Tracheal swabs | | | | | Cloacal swabs | | | | |
| **Group** | **2 days p.c.** | **4 days p.c.** | **7 days p.c.** | **10 days p.c.** | **14 days p.c.** | **2 days p.c.** | **4 days p.c.** | **7 days p.c.** | **10 days p.c.** | **14 days p.c.** |
| **2 (vaccinated twice)** | 1*/13^{a} | 0/10 | 0/8 | 0/8 | 0/8 | 1*/13 | 0/10 | 0/8 | 0/8 | 0/8 |
| | | | | | | | | | | |
| **3 (vaccinated once)** | 1/13 | 3**/11 | 0/8 | 0/8 | 0/8 | 1/13 | 2/11 | 2/8 | 0/8 | 0/8 |
| | | | | | | | | | | |
| **Control** | 7/8 | - | - | - | - | 8/8 | - | - | - | - |

| | | | | | | | | | | |
|---|---|---|---|---|---|---|---|---|---|---|
| ^{a} Number of chickens positive/number of chickens analysed ^{*} Subsequently died of AI ** Includes one bird which died of AI | | | | | | | | | | |

**Table 4. Results of the iIFAT validation on field sera. Total number of turkey serum samples tested: 608.**

| | | **iIFAT positive** | **iIFAT negative** |
|---|---|---|---|
| Number of sera from animals naturally infected with H7N1 (HI titre ≥1:16)* | 107 | 105 | 2 |
| Number of sera from H7 negative animals | 140 | 6 | 134 |
| Number of sera from H7N3 vaccinated animals | 361 | 18 | 342 |

SEQUENCE LISTING

Individual Applicant

----------------

Street :

City :

State :

Country :

PostalCode :

PhoneNumber :

FaxNumber :

EmailAddress :

<110> LastName : CAPUA

<110> FirstName : Ilaria

<110> MiddleInitial :

<110> Suffix :

Individual Applicant

----------------

Street :

City :

State :

Country :

PostalCode :

PhoneNumber :

FaxNumber :

EmailAddress :

<110> LastName : MARANGON

<110> FirstName : Stefano

<110> MiddleInitial :

<110> Suffix :

Individual Applicant

---------------

Street :

City :

State :

Country :

PostalCode :

PhoneNumber :

FaxNumber :

EmailAddress :

<110> LastName : CATTOLI

<110> FirstName : Giovanni

<110> MiddleInitial :

<110> Suffix :

Application Project

---------------

<120> Title : Purified subfragment codifyng for neiroaminidase, recombinant neuroaminidase and its

use in zooprophylaxis

<130> AppFileReference : E050462-LG

<140> CurrentAppNumber :

<141> CurrentFilingDate : ____-__-__

Sequence
-------
<213> OrganismName :
<400> PreSequenceString :
gcgcgcggcc gccaggagtt taaaatgaat ccaaatc 37
<212> Type : DNA
<211> Length : 37
SequenceName : seq1
SequenceDescription :
Custom Codon
----------
Sequence Name : SEQ. I.D. No. 1

Sequence
-------
<213> OrganismName :
<400> PreSequenceString :
gcgcgcggcc gcctacttgt caatggtgaa tggc 34
<212> Type : DNA
<211> Length : 34
SequenceName : SEQ. I.D. No. 2
SequenceDescription :
Custom Codon
----------
Sequence Name : SEQ. I.D. No. 2

## Claims

1. Use of an inactivated avian influenza virus in the preparation of a medicament for protecting against infection with avian influenza virus, wherein the inactivated virus is **characterized by** the same subtype of viral haemagglutinin and a different subtype of neuraminidase compared to the infectious virus against which protection is sought.

2. The use of Claim 1 wherein the avian influenza virus against which protection is sought is a highly pathogenic form (HPAI).

3. The use of Claim 1 wherein the avian influenza virus against which protection is sought is a low pathogenic form (LPAI).

4. The use according to any one of the preceding claims wherein the inactivated avian influenza virus is an inactivated natural virus.

5. A purified subfragment, obtainable by means of a polymerase-based amplification process with reverse transcriptase RT-PCR, of an RNA sequence coding for a neuroaminidase protein NAy derived from the genome of an avian influenza virus with epidemic subtype (HxNy).

6. A purified subfragment according to claim 5, in which said neuroaminidase protein NAy is represented by NA1, and in which said reverse transcriptase RT-PCR polymerises a cDNA derived from said sequence, said cDNA being linked to the following two polynucleotide sequences: forward primer 5'- GCG CGC GGC CGC CAG GAG TTT AAA ATG AAT CCA AAT C -3' and reverse primer 5'-GCG CGC GGC CGC CTA CTT GTC AAT GGT GAA TGG C -3'.

7. A purified subfragment according to claim 5, which has a length of about 1.4 Kb.

8. A plasmid comprising the subfragment according to one or more of the preceding claims.

9. A process for expressing a neuroaminidase protein NAy, in particular for use as an antigenic marker for tests for the detection of anti-NAy antibodies induced by avian influenza viruses with epidemic subtype (HxNy), comprising a step of expression in baculovirus vector of a cDNA coding for said protein.

10. A process according to claim 9, comprising:- a step of cloning of said baculovirus vector in E.coli cells; - a step of infection of insect cells, especially Trichoplusia ni, by means of said baculovirus vector.

11. A process according to claim 9, in which said step of expression in baculovirus vector occurs in an expression system Bac-to-Bac®.

12. A process according to claim 9, in which said neuroaminidase protein NAy to be expressed is derivable from the subfragment of claim 5 or claim 6.

13. A process according to claim 12, in which said subfragment is digested with a restriction enzyme NotI, linked to a donor plasmid pFast-Bac cleaved with said restriction enzyme NotI and cloned into competent E.coli DH5α competent cells.

14. A process according to claim 13, in which E.coli DH10Bac cells derived from said process and containing said baculovirus plasmid are pFast-Bac-transformed, said plasmid is isolated from said cells and used for the transfection of Trichoplusia ni insect cells.

15. A recombinant antigen obtainable by expression in baculovirus vector of the subfragment of claim 5 or claim 6.

16. A recombinant antigen according to claim 15, comprising a cellular carrier having artificially incorporated said recombinant baculovirus.

17. A recombinant antigen according to claim 15, comprising an insect cell carrier, especially Trichoplusia ni, infected with said recombinant baculovirus cloned in an E.coli bacterium.

18. A recombinant antigen according to any of claims 15 to 17, in which said subfragment can be expressed by means of the expression process of one or more of claims 9 to 14.

19. A recombinant antigen according to claim 15, in which said antigen consists of a purified protein obtained from said subfragment.

20. A recombinant antigen with at least one genomic sequence coding for a neuroaminidase protein NAy, for use in a test for the detection of anti-NAy antibodies induced by avian influenza virus with specific epidemic strain (HxNy).

21. A recombinant antigen according to claim 20, in which said detection test is carried out on a biological fluid of animals selected from a group of animals at least a part of which have been subjected to vaccination by means of a heterologous vaccine **characterized by** the same subtype of viral haemoagglutinin Hax and a different subtype of neuroaminidase NAy.

22. A recombinant antigen according to claim 20 or 21, in which said genomic sequence is obtainable by expression in baculovirus of the subfragment of claim 5 or claim 6.

23. A recombinant antigen according to claim 20 or 21, in which said genomic sequence coding for a neuroaminidase protein NAy can be expressed by means of the expression process of one or more of claims 9 to 14.

24. A diagnostic method for detecting the positivity to an avian influenza virus infection with specific epidemic strain (HxNy) comprising the steps of:
- preparing an antigen having at least one genomic sequence coding for a neuroaminidase protein (NAy);
- contacting said antigen with a specimen of animal biological fluid to be tested;
- evidencing of an antigen-antibody reaction by means of a positivity detection test.

25. A diagnostic method according to claim 24, in which said test for the detection of positivity is an immunofluorescence or immunoperoxidase test.

26. A diagnostic method according to claim 24, in which said test for the detection of positivity is an ELISA test.

27. A diagnostic method according to claim 24, in which said test for the detection of positivity is a colour test that is adapted to be carried out on the field by means of an inert support with said antigen adsorbed on.

28. A process for the vaccination against avian influenza virus infection with specific epidemic strain HxNy comprising the steps of:
- preparing a heterologous vaccine **characterized by** the same subtype of viral haemoagglutinin Hax and a different subtype of neuroaminidase Naz;
- administering said vaccine to at least one group of animals selected from a population at risk of infection;
- detecting the positivity to the virus infection of said avian influenza by: - preparing an antigen having at least one genomic sequence coding for a neuroaminidase protein (NAy); taking biological fluids from the body of the animals comprised in said animal population; contacting said antigen with said biological fluids, with subsequent evidencing of an antigen-antibody reaction by means of a positivity detection test.

29. A vaccination process according to claim 28, in which said detection step is obtained by means of the diagnostic method according to one or more of claims 24 to 27.

30. A vaccination process according to claim 28, in which said antigen is obtained according to one or more of claims 15 to 19.

31. A vaccination process according to claim 28, in which said vaccine is a natural vaccine obtained by inactivating a natural virus.

32. A diagnostic kit for a test for detecting on an animal population the positivity to an avian influenza virus infection with epidemic subtype (HxNy), comprising
- a solid support of an inert material;
- an antigen with at least a genomic sequence coding for a neuroaminidase protein NAy in a state that is substantially non modified as compared with that of the specific avian influenza virus strain (H×Ny), said antigen being associated onto said solid support;
- a reagent that is adapted to colorimetrically evidence the positivity to infection in the presence of anti-NAy antibodies contained in a biological fluid of an animal.

33. A diagnostic kit according to claim 32, in which said biological fluid is derived from a population of animals at risk of infection, at least one group thereof having been treated with a heterologous vaccine **characterized by** the same subtype of viral haemoagglutinin Hax and a different subtype of neuroaminidase Naz, said kit establishing in any case the discrimination of the infected individuals from the other individuals.

34. A diagnostic kit according to claim 28, in which said antigen is obtained in accordance with one or more of claims 15 to 19.

35. A diagnostic kit according to claim 32, in which said support is selected from the group comprising: latex spheres, plastic supports.
